(19) Europäisches Patentamt
European Patent Office
Office européen des brevets

(11) **EP 3 134 394 B1**

(12) **EUROPÄISCHE PATENTSCHRIFT**

(45) Veröffentlichungstag und Bekanntmachung des Hinweises auf die Patenterteilung:
**28.02.2018 Patentblatt 2018/09**

(21) Anmeldenummer: **15721149.1**

(22) Anmeldetag: **23.04.2015**

(51) Int Cl.:
*C07D 307/89* (2006.01)     *B01J 23/22* (2006.01)
*B01J 8/04* (2006.01)     *B01J 8/06* (2006.01)
*B01J 21/06* (2006.01)     *B01J 27/198* (2006.01)
*B01J 35/02* (2006.01)     *B01J 35/10* (2006.01)

(86) Internationale Anmeldenummer:
**PCT/EP2015/058854**

(87) Internationale Veröffentlichungsnummer:
**WO 2015/162230 (29.10.2015 Gazette 2015/43)**

(54) **KATALYSATORANORDNUNG MIT OPTIMIERTER OBERFLÄCHE ZUR HERSTELLUNG VON PHTHALSÄUREANHYDRID**

CATALYTIC CONVERTER ARRANGEMENT WITH OPTIMIZED SURFACE FOR PRODUCING PHTHALIC ANHYDRIDE

DISPOSITIF CATALYTIQUE COMPRENANT UNE SURFACE OPTIMISÉE POUR LA PRODUCTION D'ANHYDRIDE D'ACIDE PHTALIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.04.2014 DE 102014005939**

(43) Veröffentlichungstag der Anmeldung:
**01.03.2017 Patentblatt 2017/09**

(73) Patentinhaber: **Clariant International Ltd**
**4132 Muttenz (CH)**

(72) Erfinder:
• **RICHTER, Oliver**
**82110 Germering (DE)**
• **MESTL, Gerhard**
**80935 München (DE)**
• **SCHULZ, Felix**
**81373 München (DE)**
• **PITSCHI, Werner**
**83052 Bruckmühl (DE)**
• **FROMM, Nadine**
**93109 Grosskarolinenfeld (DE)**
• **SCHINKE, Peter**
**80804 München (DE)**

(74) Vertreter: **Silber, Anton**
**Clariant Produkte (Deutschland) GmbH**
**Patent & License Management**
**Lenbachplatz 6**
**80333 München (DE)**

(56) Entgegenhaltungen:
**EP-A1- 0 985 648     WO-A1-2008/077791**

**Beschreibung**

Die Erfindung betrifft eine Katalysatoranordnung zur Herstellung von Phthalsäureanhydrid durch Gasphasenoxidation aromatischer Kohlenwasserstoffe, umfassend einen Reaktor mit einer Gaseintrittsseite für ein Eduktgas und einer Gasaustrittsseite für ein Produktgas, sowie einer ersten Katalysatorlage aus Katalysatorkörpern, und mindestens einer zweiten Katalysatorlage aus Katalysatorkörpern, wobei die erste Katalysatorlage an der Gaseintrittsseite und die zweite Katalysatorlage nachfolgend zur ersten Katalysatorlage in Gasdurchflussrichtung angeordnet ist und die Katalysatorkörper eine äußere Schicht Aktivmasse aufweisen, dadurch gekennzeichnet, dass in der ersten Katalysatorlage und/oder in der zweiten Katalysatorlage der Aktivmasse-gehalt, bezogen auf das Gesamtgewicht der Katalysatorkörper, unter 7 Gew.-% beträgt und in der jeweiligen Katalysatorlage das Verhältnis der Gesamtoberfläche der Aktivmasse zum Volumen der Katalysatorlage bevorzugt 10000 cm$^{-1}$ bis 20000 cm$^{-1}$, besonders bevorzugt 12000 cm$^{-1}$ bis 16000 cm$^{-1}$ beträgt.

Die großtechnische Produktion von Phthalsäureanhydrid erfolgt durch katalytische Gasphasenoxidation von ortho-Xylol und / oder Naphthalin. Zu diesem Zweck wird ein für die Reaktion geeigneter Katalysator, im Allgemeinen ein vanadiumhaltiger Katalysator, in einem Reaktor bereitgestellt und ein Reaktionsgas über den Katalysator geleitet. Vorzugsweise verwendet man als Reaktor einen sogenannten Rohrbündelreaktor, in dem eine Vielzahl von Rohren parallel angeordnet sind und die von einem Kühlmittel umströmt werden. Als Kühlmittel verwendet man im Allgemeinen eine Salzschmelze, beispielsweise ein eutektisches Gemisch aus $NaNO_2$ und $KNO_3$. Der Katalysator wird in Form von Katalysatorkörpern in die Rohre eingefüllt. Im einfachsten Fall verwendet man eine homogene Schüttung. Über die Schüttung wird dann ein Reaktionsgas geleitet, welches ein Gemisch aus einem sauerstoffhaltigen Gas, meist Luft, und dem zu oxidierenden Kohlenwasserstoff, meist ortho-Xylol oder Naphthalin, enthält.

Heutzutage werden industrielle Phthalsäureanhydrid-Katalysatoren auf Basis von $V_2O_5$-$TiO_2$ -haltigen Aktiv-massen verwendet, die als Schicht auf Trägerringen - meist Steatit - aufgetragen werden. Die Oxidation des Kohlenwasserstoffs ist stark exotherm, so dass insbesondere im Bereich des Reaktoreingangs eine starke Wärmeentwicklung zu beobachten ist, die zur Totaloxidation des Kohlenwasserstoffs und zur Deaktivierung des Katalysators führen kann. Um den damit einhergehenden Produktivitätsverlust zu vermeiden, ist man dazu übergegangen, strukturierte Katalysator-Schüttungen, d.h. Katalysatoranordnungen, zu verwenden, wobei in den Rohren Lagen aus Katalysatoren unterschiedlicher Aktivität übereinander angeordnet sind. Gegenwärtig werden meist drei- oder vierlagige Katalysator-Schüttungen verwendet, wobei auf der Seite des Reaktoreingangs eine erste Katalysatorlage mit relativ niedriger Aktivität angeordnet ist, auf welche dann Katalysatorlagen mit schrittweise erhöhter Aktivität folgen. Die Katalysatorlage mit der höchsten Aktivität ist also auf der Seite des Reaktorausgangs angeordnet. Solche Systeme sind beispielsweise aus den Schriften WO 99/61434 A1, WO99/61433 A1 oder WO 2004/103944 A1 bekannt.

In jüngerer Zeit ist man dazu übergegangen, vermehrt Katalysatorsysteme mit vier oder mehr Lagen zu verwenden, wobei an der Reaktoreingangsseite zunächst eine relativ kurze Lage eines Katalysators höherer Aktivität angeordnet ist. An dieser Lage höherer Aktivität schließt sich eine Lage mit niedriger Aktivität an, die von weiteren Lagen gefolgt wird, bei denen sich die Katalysatoraktivität wieder erhöht. Solche Katalysatorsysteme sind beispielsweise aus den Schriften WO 2007/134849 A1 und WO 2011/032658 A1 bekannt.

WO 2006/092304 A1 beschreibt die Verwendung eines Katalysators enthaltend mindestens eine erste zur Gaseintrittsseite hin gelegene Katalysatorlage, eine zweite, näher zur Gasaustrittseite hin gelegene Katalysatorlage und eine dritte, noch näher zur oder an der Gasaustrittsseite hin gelegene Katalysatorlage zur Herstellung von Phthalsäureanhydrid durch Gasphasenoxidation von o-Xylol und/oder Naphthalin, wobei die Katalysatorlagen vorzugsweise jeweils eine Aktivmasse enthaltend $TiO_2$ aufweisen, dadurch gekennzeichnet, dass die Katalysatoraktivität der ersten Katalysatorlage höher ist, als die Katalysatoraktivität der zweiten Katalysatorlage. Die Aktivität der ersten Katalysatorlage kann durch alle dem Fachmann geläufigen Maßnahmen so eingestellt werden, dass sie höher liegt als die Aktivität der nachfolgenden zweiten Katalysatorlage. Nach einer bevorzugten Ausführungsform kann die erhöhte Aktivität in der ersten Katalysatorlage beispielsweise erzielt werden durch eine Erhöhung der Schüttdichte in der ersten Katalysatorlage, z.B. durch Einsatz einer anderen (Ring)geometrie des verwendeten inerten Formkörpers.

WO 2008/077791 A1 beschreibt ein Verfahren zur Gasphasenoxidation, bei dem man einen gasförmigen Strom, der einen aromatischen Kohlenwasserstoff und molekularen Sauerstoff umfasst, durch zwei oder mehrere Katalysatorlagen leitet. Ferner betrifft diese Offenlegungsschrift ein Katalysatorsystem zur Gasphasenreaktion unter Verwendung einer Vorlage. Das Produkt aus Durchmesser mal Höhe, oder das Volumen der vorgelagerten Inert- und/oder Katalysatoren-Ringe, ist kleiner als mindestens einer der folgenden Katalysatorlagen oder der Quotient aus Oberfläche pro Volumen der vorgelagerten Inert- und/oder Katalysatoren-Ringe ist größer als mindestens einer der folgenden Katalysatorlagen.

EP 0985648 A1 betrifft die Gasphasenoxidation von Kohlenwasserstoffen, bei der eine gasförmige Mischung umfassend ein molekularen Sauerstoff enthaltendes Gas und Kohlenwasserstoffe, die Substituenten enthalten können, durch ein Katalysatorfestbett geleitet wird und stellt ein Verfahren zur Gasphasenoxidation zur Verfügung, das durchgeführt wird, indem eine gasförmige Mischung von Rohmaterialien durch ein Katalysatorfestbett geleitet wird, in dem sich der Hohlraumanteil der Katalysatorschichten in einem oder mehreren Schritten in Strömungsrichtung entlang des Flusses der gasförmigen Mischung der Rohmaterialien erhöht.

Bei der Oxidation von ortho-Xylol oder Naphthalin entstehen neben dem Wertprodukt Phthalsäureanhydrid noch eine Reihe unerwünschter Nebenprodukte, die durch unvollständige Oxidation oder durch Überoxidation entstehen. Nebenprodukte, die durch unvollständige Oxidation entstehen, sind hauptsächlich ortho-Tolylaldehyd und Phthalid. Nebenprodukte, die durch Überoxidation entstehen, sind hauptsächlich Kohlenmonoxid, Kohlendioxid und Maleinsäureanhydrid, neben geringeren Mengen an Benzoesäure und Citraconsäureanhydrid. Gewünscht wird eine möglichst hohe Selektivität der Oxidation zu Phthalsäureanhydrid bei möglichst geringen Anteilen von Nebenprodukten im Endprodukt, darunter insbesondere Maleinsäureanhydrid, bei gleichzeitigem hohem Umsatz des Ausgangsprodukts.

Gegenwärtig werden molare Selektivitäten an Phthalsäureanhydrid von bis zu 83 mol.-% erreicht. Um die Selektivität der Oxidation von ortho-Xylol bzw. Naphthalin zu Phthalsäureanhydrid weiter zu erhöhen, können verschiedene Parameter des Katalysatorsystems variiert werden. So kann die Zusammensetzung des Katalysators variiert werden oder auch die Eigenschaften der Schüttung des Katalysators. Dazu kann beispielsweise die Anordnung und die Länge der einzelnen Katalysatorlagen variiert werden.

Der Erfindung liegt die Aufgabe zugrunde, eine Katalysatoranordnung zur Herstellung von Phthalsäureanhydrid durch Gasphasenoxidation aromatischer Kohlenwasserstoffe bereitzustellen, die im Vergleich zu den aus dem Stand der Technik bekannten Katalysatoranordnungen eine erhöhte Ausbeute an Phthalsäureanhydrid ermöglicht und den Erhalt von roh-Phthalsäureanhydrid mit einem höheren Reinheitsgrad.

Die Aufgabe der Erfindung wird gelöst mit einer Katalysatoranordnung zur Herstellung von Phthalsäureanhydrid durch Gasphasenoxidation aromatischer Kohlenwasserstoffe, umfassend einen Reaktor mit einer Gaseintrittsseite für ein Eduktgas und einer Gasaustrittsseite für ein Produktgas, sowie einer ersten Katalysatorlage aus Katalysatorkörpern, und mindestens einer zweiten Katalysatorlage aus Katalysatorkörpern, wobei die erste Katalysatorlage an der Gaseintrittsseite und die zweite Katalysatorlage nachfolgend zur ersten Katalysatorlage in Gasdurchflussrichtung angeordnet ist und die Katalysatorkörper eine äußere Schicht Aktivmasse aufweisen, dadurch gekennzeichnet, dass in der ersten Katalysatorlage und/oder in der zweiten Katalysatorlage der Aktivmassegehalt, bezogen auf das Gesamtgewicht der Katalysatorkörper, unter 7 Gew.-% beträgt und in der jeweiligen Katalysatorlage das Verhältnis der Gesamtoberfläche der Aktivmasse zum Volumen der Katalysatorlage bevorzugt 10000 cm$^{-1}$ bis 20000 cm$^{-1}$, besonders bevorzugt 12000 cm$^{-1}$ bis 16000 cm$^{-1}$ beträgt.

Das erfindungsgemäße Verfahren wird in einem oder mehreren Reaktor(en) durchgeführt, wobei der oder die Reaktor(en) vorzugweise röhrenförmig sind (Reaktorrohre oder Rohre). Die Öffnungen des Reaktors, typischerweise die beiden Öffnungen des Reaktorrohrs, bilden einen Reaktoreingang für das Eduktgas sowie einen Reaktorausgang für das Produktgas, so dass eine Gaseintrittsseite, eine Gasaustrittseite und eine Gasdurchflussrichtung vorhanden sind. Der Reaktor wird z.B. in einem Salzbad auf eine Temperatur von zwischen 340°C und 450°C erhitzt, wobei sich durch die exotherme Reakt ion und durch die eventuell vorhandenen verschiedenen Katalysatorlagen ein Temperaturprofil ausbildet.

Der vorzugsweise röhrenförmige Reaktor weist jeweils einen Durchmesser (D) auf, wobei hier auf den Innendurchmesser des Reaktors Bezug genommen wird. Bevorzugt wird ein Durchmesser (D) des Reaktors im Bereich von 10 bis 50 mm, weiter bevorzugt 20 bis 40 mm gewählt. Die röhrenförmigen Reaktoren weisen eine Rohrlänge (L) auf, welche ebenfalls in üblichen Bereichen gewählt wird, so z.B. 2 bis 5 m. Die Rohrlänge L entspricht dabei dem Anteil der Länge des Reaktorrohrs, welcher mit den Katalysatorkörpern gefüllt ist.

Der Reaktor wird jeweils mit Katalysatorkörpern gefüllt, die die Gasphasenoxidation des ortho-Xylols oder Naphthalins katalysieren. Die Katalysatorkörper bestehen üblicherweise aus einem inerten Trägerkörper, der mit einer vanadiumhaltigen Aktivmasse, d.h. der katalytisch aktiven Masse, beladen ist. Erfindungsgemäß müssen mindestens zwei Katalysatorlagen ausgebildet werden, wobei eine Katalysatorlage eine einheitliche Schüttung von Katalysatorkörpern im Reaktor darstellt. Die Ausdehnung der Schüttung im Reaktor in axialer Richtung bzw. in Gasdurchflussrichtung entspricht dabei der Länge $L_x$ der jeweiligen Katalysatorlage x. Sofern der Reaktor als senkrecht angeordnetes Rohr ausgebildet ist, ist die Länge der jeweiligen Katalysatorlage gleichbedeutend mit der Füllhöhe der jeweiligen Katalysatorlage. Die Zählung der x-ten Katalysatorlage erfolgt von der Gaseintrittsseite in Gasdurchflussrichtung. Um die Reaktion durchzuführen, werden durch den Reaktor z.B. stündlich von oben nach unten etwa 2 bis 5 Nm$^3$ Luft mit einer Beladung von 30 bis 100 g ortho-Xylol/Nm$^3$ Luft bei einem Gesamtdruck von ca. 1,2 bis 1,6 bar geleitet. Generell können die aufeinander folgenden Katalysatorlagen unmittelbar aufeinander folgen, d.h. in Kontakt stehen, oder aber, z.B. durch eine Lage Inertkörper, voneinander getrennt vorliegen.

Die erste Katalysatorlage ist der Gaseintrittsseite des Reaktors zugewandt, unmittelbar daran anschließend in Gasdurchflussrichtung befindet sich die mindestens zweite Katalysatorlage, bestehend aus Katalysatorkörpern, die sich von den Katalysatorkörpern der ersten Katalysatorlage unterscheiden. Erfindungsgemäß kann die erste Katalysatorlage einen höheren Lückengrad aufweisen als die zweite Katalysatorlage.

Die erste Katalysatorlage kann eine Länge von kleiner 1 m, bevorzugterweise von 10 bis 50 cm aufweisen. Es wird bevorzugt, dass die erste Katalysatorlage einen Längenanteil von 5 bis 25 %, besonders bevorzugt 15 bis 25 % der Rohrlänge L aufweist.

Die zweite Katalysatorlage kann eine Länge von 0,3 bis 3 m bevorzugterweise von 0,85 bis 2 m, besonders bevorzugterweise 1 bis 2 m aufweisen. Es wird bevorzugt, dass die zweite Katalysatorlage einen Längenanteil von 15 bis 60 %, insbesondere 20 bis 60 % oder 30 bis 50 % der Rohrlänge L aufweist.

An die zweite Katalysatorlage können sich in Gasdurchflussrichtung weitere Katalysatorlagen anschließen, z.B. eine dritte Katalysatorlage, die sich unmittelbar an die zweite Katalysatorlage anschließt, und möglicherweise eine vierte Katalysatorlage, die sich unmittelbar an die dritte Katalysatorlage anschließt. Eine entsprechende fünfte Katalysatorlage ist in der Regel nicht erforderlich, jedoch möglich.

Die dritte Katalysatorlage kann eine Länge von kleiner 1 m, bevorzugterweise 50 bis 70 cm aufweisen. Es wird bevorzugt, dass die dritte Katalysatorlage einen Längenanteil von etwa 10 bis 30 % der Rohrlänge L einnimmt, insbesondere wenn es sich bei der dritten Katalysatorlage um die letzte, also die dem Reaktorausgang am nächsten gelegene Katalysatorlage handelt, ist ein Längenanteil für die dritte Katalysatorlage von 20 bis 50 % der Rohrlänge L bevorzugt.

Die vierte Katalysatorlage kann eine Länge von 30 bis 150 cm, bevorzugterweise 55 bis 75 cm aufweisen. Es wird bevorzugt, dass eine solche vierte Katalysatorlage einen Längenanteil von etwa 10 bis 40 %, insbesondere bevorzugt 10 bis 30 % der Rohrlänge L einnimmt, insbesondere wenn es sich bei der vierten Katalysatorlage um die letzte, also die dem Reaktorausgang am nächsten gelegene Katalysatorlage handelt, ist ein Längenanteil an der Rohrlänge L für die vierte Katalysatorlage von 15 bis 25 % bevorzugt.

In einer weiteren besonders bevorzugten Ausführungsform ist der Längenanteil an der Rohrlänge L der ersten Katalysatorlage zwischen 15 bis 25 %, der zweiten Katalysatorlage zwischen 20 bis 50 %, der dritten Katalysatorlage zwischen 20 bis 50 % und der vierten Katalysatorlage zwischen 15 bis 25 %.

Gemäß der Erfindung beträgt für die erste und / oder für die zweite Katalysatorlage das Verhältnis der Gesamtoberfläche der Aktivmasse zum Volumen der Katalysatorlage ($OF_A / V_x$) bevorzugt 10000 cm$^{-1}$ bis 20000 cm$^{-1}$, besonders bevorzugt 12000 cm$^{-1}$ bis 16000 cm$^{-1}$. Die Gesamtoberfläche der Aktivmasse $OF_A$ berechnet sich nach Gl. 1:

Gl. 1:

$$OF_A = A_A \times m_x \times BET_A$$

$OF_A$ = Gesamtoberfläche der Aktivmasse
$m_x$ = Gesamtmasse der Katalysatorkörper in der Katalysatorlage
$BET_A$ = spezifische Oberfläche (BET, Einheit [m$^2$/g]) der Aktivmasse in der jeweiligen Katalysatorlage

Die Gesamtmasse kann entweder durch Abwiegen oder über die Schüttdichte nach Gl. 2 bestimmt werden:

Gl. 2:

$$m_x = \delta_{Sch\ddot{u}tt} \times V_x$$

$\delta_{Srh\ddot{u}tt}$ = Schüttdichte der jeweiligen Katalysatorlage
$V_x$ = Volumen $V_x$ der Katalysatorlage

Das Volumen $V_x$ der Katalysatorlage berechnet sich nach Gl. 3:

Gl. 3:

$$V_x = \frac{D^2}{4} \times \pi \times L_x$$

$V_x$ = Volumen der Katalysatorlage x
$D$ = Durchmesser des Reaktors
$L_x$ = Länge der Katalysatorlage x

Das Verhältnis der Gesamtoberfläche der Aktivmasse zum Volumen der Katalysatorlage zwischen der ersten Katalysatorlage und der zweiten Katalysatorlage kann sich um mehr als 5 % oder um mehr als 7 % unterscheiden.

Die Katalysatorkörper der unterschiedlichen Katalysatorlagen können sich auch im Aktivmassegehalt unterscheiden. Erfindungsgemäß weisen die Katalysatorkörper der ersten und / oder der zweiten Katalysatorlage einen Aktivmassegehalt zwischen 1 bis 7 Gew.-%, bevorzugt zwischen 2 bis 6 Gew.-%, besonders bevorzugt zwischen 3 bis 5 Gew.-% und stärker bevorzugt unter 4 Gew.-% auf, bezogen auf das Gesamtgewicht der Katalysatorkörper. Erfindungsgemäß weisen die Katalysatorkörper der dritten und / oder der vierten Katalysatorlage einen Aktivmassegehalt zwischen 3 bis 12 Gew.-%, bevorzugt zwischen 4 bis 10 Gew.-% auf, bezogen auf das Gesamtgewicht der Katalysatorkörper.

Bevorzugt ist, dass der Aktivmassegehalt der ersten Katalysatorlage höher ist, als der der zweiten Katalysatorlage. Z.B. kann der Aktivmassegehalt der Katalysatorkörper der ersten Katalysatorlage bei 7 bis 9 Gew.-% liegen und der der zweiten Katalysatorlage bei 2 bis 6 Gew.-%, jeweils bezogen auf das Gesamtgewicht der Katalysatorkörper. Bezüglich der möglicherweise folgenden dritten und vierten Katalysatorlage ist bevorzugt, dass sich der Aktivmassegehalt $m_A$ von der zweiten zur vierten Katalysatorlage erhöht, oder sich von der zweiten auf die dritte Katalysatorlage erhöht und von der dritten auf die vierte Katalysatorlage gleichbleibt.

Die Aktivmasse kann neben Vanadium zahlreiche Promotoren wie beispielsweise Alkali- und / oder Erdalkalimetalle, Antimon, Phosphor, Eisen, Niob, Kobalt, Molybdän, Silber, Wolfram, Zinn, Blei, Zirkonium, Kupfer, Gold, und/oder Wismut sowie Mischungen aus zwei oder mehreren der vorstehenden Komponenten haben. Die Katalysatorkörper der einzelnen Lagen unterscheiden sich in einer Ausführungsform in der Zusammensetzung ihrer Aktivmasse. Tabelle 1 gibt einen Überblick zur typischen Zusammensetzung der Aktivmasse.

**Tabelle 1: Typische Zusammensetzung der Aktivmasse**

| Komponente | Typischer Gehalt* |
|---|---|
| $V_2O_5$ | 0,5 bis 30 Gew.-%, insbesondere 1 bis 30 Gew.-% |
| $Sb_2O_3$ oder $Sb_2O_5$ | 0 bis 10 Gew.-% |
| Cs | 0 bis 2 Gew.-% |
| P | 0 bis 5 Gew.-% |
| Nb | 0 bis 5 Gew.-% |
| Weitere Komponenten wie Li, Na, K, Ba, W, Mo, Y, Ce, Mg, Sn, Bi, Fe, Ag, Co, Ni, Cu, Au, etc. | 0 bis 5 Gew.-% |
| $TiO_2$ | Rest zu 100 Gew.-% |
| *Die Prozentangaben beziehen sich jeweils auf das Gesamtgewicht der Aktivmasse. | |

Die Aktivmasse enthält vorzugsweise 5 bis 15 Gew.-% $V_2O_5$, 0 bis 5 Gew.-% $Sb_2O_3$, 0,2 bis 0,75 Gew.-% Cs, 0 bis 5 Gew.-% $Nb_2O_5$, jeweils bezogen auf das Gesamtgewicht der Aktivmasse. Neben den vorstehenden Komponenten besteht der Rest der Aktivmasse ganz oder im Wesentlichen aus $TiO_2$. Eine solche Aktivmasse kann beispielsweise vorteilhaft in den Katalysatorkörpern der ersten oder zweiten Katalysatorlage verwendet werden.

Bevorzugterweise weisen die Katalysatorkörper abhängig von der Katalysatorlage unterschiedlich zusammengesetzte Aktivmassen auf und/oder die Aktivmassen der einzelnen Katalysatorlagen unterscheiden sich in den physikalisch-chemischen Eigenschaften. Nach einer Ausführungsform liegt die BET-Oberfläche des Katalysators bzw. der Aktivmasse zwischen 15 und etwa 30 m$^2$/g. Die Aktivmasse kann aber z.B. in den Katalysatorkörpern abhängig von der Katalysatorlage jeweils eine unterschiedliche BET-Oberfläche aufweisen.

Nach einer bevorzugten Ausführungsform nimmt dabei die BET-Oberfläche der Aktivmasse der ersten Katalysatorlage zur Aktivmasse der vierten Katalysatorlage zu. Geeignete Bereiche für die BET-Oberfläche sind beispielsweise 15 bis 25 m$^2$/g für die erste Katalysatorlage, 15 bis 30 m$^2$/g für die zweite Katalysatorlage, 15 bis 30 m$^2$/g für die dritte Katalysatorlage und 20 bis 45 m$^2$/g für die vierte Katalysatorlage.

Nach einer besonders bevorzugten Ausführungsform enthält die Aktivmasse der Katalysatorkörper der ersten Katalysatorlage zwischen 5 bis 16 Gew.-% $V_2O_5$, 0 bis 5 Gew.-% $Sb_2O_3$, 0,2 bis 0,75 Gew.-% Cs, 0 bis 1 Gew.-% P und 0 bis 3 Gew.-% $Nb_2O_5$, jeweils bezogen auf das Gesamtgewicht der Aktivmasse. Der Rest der Aktivmasse besteht zu mindestens 90 Gew.-%, vorzugsweise mindestens 95 Gew.-%, weiter bevorzugt mindestens 98 Gew.-%, insbesondere mindestens 99 Gew.-%, weiter bevorzugt mindestens 99,5 Gew.-%, insbesondere 100 Gew.-% aus $TiO_2$. Nach einer besonders bevorzugten Ausführungsform liegt dabei die BET-Oberfläche des $TiO_2$ zwischen 15 und etwa 25 m$^2$/g.

Nach einer besonders bevorzugten Ausführungsform enthält die Aktivmasse der Katalysatorkörper der zweiten Katalysatorlage zwischen 5 bis 15 Gew.-% $V_2O_5$, 0 bis 5 Gew.-% $Sb_2O_3$, 0,2 bis 0,75 Gew.-% Cs, 0 bis 1 Gew.-% P und 0 bis 2 Gew.-% $Nb_2O_5$, jeweils bezogen auf das Gesamtgewicht der Aktivmasse. Der Rest der Aktivmasse besteht zu mindestens 90 Gew.-%, vorzugsweise mindestens 95 Gew.-%, weiter bevorzugt mindestens 98 Gew.-%, insbesondere mindestens 99 Gew.-%, weiter bevorzugt mindestens 99,5 Gew.-%, insbesondere 100 Gew.-% aus $TiO_2$. Nach einer besonders bevorzugten Ausführungsform liegt dabei die BET-Oberfläche des $TiO_2$ zwischen 15 und etwa 45 $m^2$/g.

Nach einer besonders bevorzugten Ausführungsform enthält die Aktivmasse der Katalysatorkörper der dritten Katalysatorlage zwischen 5 bis 15 Gew.-% $V_2O_5$, 0 bis 4 Gew.-% $Sb_2O_3$, 0,05 bis 0,5 Gew.-% Cs, 0 bis 1 Gew.-% P und 0 bis 2 Gew.-% $Nb_2O_5$, jeweils bezogen auf das Gesamtgewicht der Aktivmasse. Der Rest der Aktivmasse besteht zu mindestens 90 Gew.-%, vorzugsweise mindestens 95 Gew.-%, weiter bevorzugt mindestens 98 Gew.-%, insbesondere mindestens 99 Gew.-%, weiter bevorzugt mindestens 99,5 Gew.-%, insbesondere 100 Gew.-% aus $TiO_2$. Dabei wird bevorzugt, dass das $TiO_2$ eine BET-Oberfläche zwischen 15 und 25 $m^2$/g aufweist.

Nach einer besonders bevorzugten Ausführungsform enthält die Aktivmasse des Katalysators der vierten Katalysatorlage zwischen 5 bis 25 Gew.-% $V_2O_5$, 0 bis 5 Gew.-% $Sb_2O_3$, 0 bis 0,2 Gew.-% Cs, 0 bis 2 Gew.-% P und 0 bis 1 Gew.-% $Nb_2O_5$, jeweils bezogen auf das Gesamtgewicht der Aktivmasse. Der Rest der Aktivmasse besteht zu mindestens 90 Gew.-%, vorzugsweise mindestens 95 Gew.-%, weiter bevorzugt mindestens 98 Gew.-%, insbesondere mindestens 99 Gew.-%, weiter bevorzugt mindestens 99,5 Gew.-%, insbesondere 100 Gew.-% aus $TiO_2$. Soweit die vierte Katalysatorlage die an der Gasaustrittsseite des Reaktors gelegene (letzte) Katalysatorlage darstellt, wird dabei eine BET-Oberfläche des $TiO_2$ bevorzugt, die etwas höher liegt, als diejenige des $TiO_2$ der näher zur Gaseintrittsseite hin gelegenen Katalysatorlagen. Es wird bevorzugt, dass das $TiO_2$ der Aktivmasse der vierten Katalysatorlage im Bereich zwischen etwa 15 bis etwa 45 $m^2$/g liegt.

Weiterhin können sich die Katalysatorkörper der einzelnen Katalysatorlagen in ihrer intrinsischen Aktivität unterscheiden, die unterschiedliche intrinsische Aktivität kann sich zum Beispiel durch eine unterschiedliche Zusammensetzung der Aktivmasse ergeben. Erfindungsgemäß ist es bevorzugt, dass die Katalysatorkörper der ersten Katalysatorlage eine höhere intrinsische Aktivität aufweisen als die Katalysatorkörper der zweiten Katalysatorlage.

Unter der intrinsischen Aktivität eines Katalysatorkörpers $K_b$ wird die Aktivität der Aktivmasse für eine bestimmte Reaktion unter Bedingungen verstanden, unter denen die Reaktion an einem infinitesimal kleinen Katalysatorpartikel abläuft und die Reaktion durch die Nachbarschaft unbeeinflusst ist. Solche Bedingungen würden einem Temperaturgradienten über den Reaktor von 0 °C, einer unendlichen Raum-Zeit-Gesch windigkeit des Gases und einer unendlichen Entfernung zwischen den infinitesimal kleinen Katalysatorpartikeln entsprechen. Eine solche intrinsische Aktivität lässt sich beispielsweise durch eine Versuchsreihe ermitteln, die es ermöglicht, die Aktivität des Katalysatorkörpers in einem Zustand zu extrapolieren, der einem solchen idealen Zustand entspricht (z.B. Extrapolation der Versuchsergebnisse gegen einen Umsatz von Null).

Bei der praktischen Durchführung der Ermittlung der intrinsischen Aktivität wird gemäß einer Ausführungsform so vorgegangen, dass ein Katalysatorkörper mit einer bestimmten Geometrie und einem bestimmten Gehalt an Aktivmasse hergestellt wird. Ferner wird angenommen, dass die Reaktion erster Ordnung abläuft, unabhängig von der Ordnung, mit welcher die Reaktion tatsächlich abläuft. Dieser standardisierte Katalysatorkörper wird dann mit inerten Körpern so weit verdünnt, dass die Temperaturdifferenz zwischen der Gaseintritts- und der Gasaustrittsseite geringer als 25 °C, bevorzugt geringer als 10 °C ist, die Reaktion also unter nahezu isothermen Bedingungen abläuft, wobei der Druckabfall über den Reaktor weniger als 30 mbar, bevorzugt weniger als 10 mbar beträgt und wobei der Umsatz auf einen Wert im Bereich von 65 bis 95 % eingestellt wird.

Dazu wird der Katalysatorkörper mit Inertkörpern verdünnt. Die Geometrie der Katalysatorkörper und der Inertkörper wird so gewählt, dass der geforderte geringe Druckabfall verwirklicht wird. Das Verhältnis von Inertkörpern zu Katalysatorkörpern wird so gewählt, dass der geforderte Umsatz erreicht wird und gleichzeitig die Wärmeentwicklung so gering ist, dass die geforderte geringe Temperaturdifferenz zwischen Gaseintritt und Gasaustritt eingehalten wird. Bezogen auf das Volumen, welches sich aus der Schüttdichte der Katalysatorkörper bzw. der Inertkörper ergibt, wird bevorzugt ein Verhältnis von Katalysatorkörpern zu Inertkörpern von 1 : 5 bis 1 : 10 gewählt. Die Abmessungen des Testreaktors werden je nach der betrachteten Reaktion zwischen 1 und 6 m für die Länge und zwischen 18 und 32 mm für den Durchmesser des Reaktors gewählt. Bei schnellen Reaktionen wird eine kurze Länge gewählt, während für langsam ablaufende Reaktionen eine größere Reaktionsstrecke benötigt wird, um die geforderten Umsätze zu erreichen.

Es wird dann bei bestimmten Raum-Zeit-Geschwindigkeiten und bei verschiedenen bestimmten Temperaturen der Umsatz der Reaktion gemessen, die von der Aktivmasse des Katalysatorkörpers katalysiert wird. Aus den Umsätzen lässt sich dann die aktivmassenbezogene Aktivitätskonstante A* des Katalysators in Abhängigkeit vom Umsatz nach Gl. 4 berechnen:

Gl. 4:

$$A^* = \frac{[\text{GHSV} \times -1 \times \ln(1 - U)]}{[m_{\text{Aktivmasse}}]}$$

Hierbei bedeutet:

A*: Aktivmassenbezogene Aktivitätskonstante der Aktivmasse bei einer bestimmten Temperatur und GHSV;
GHSV: Raum-Zeitgeschwindigkeit [h$^{-1}$]
$m_{Aktivmasse}$: Menge der im Reaktor eingebrachten Aktivmasse [g];
U: Umsatz des Edukts, wobei sich U nach Gl. 5 berechnet.

Gl. 5:

$$U = \frac{M_{rein} - M_{raus}}{M_{rein}}$$

$M_{rein}$: Menge an Edukt [mol] welches der Katalysatorfüllung zugeführt wird
$M_{raus}$: Menge an Edukt [mol] welches die Katalysatorfüllung verlässt

Aus den ermittelten aktivmassenbezogenen Aktivitätskonstanten A* als Funktion des Umsatzes U wird dann durch lineare Extrapolation gegen einen Umsatz von Null die intrinsische Aktivität $A_{ib}$ des Katalysators bestimmt. Die für die Extrapolation einzustellenden Umsätze werden so gewählt, dass diese im linearen Bereich der Abhängigkeit der aktivmassenbezogenen Aktivitätskonstante vom Umsatz liegen (z.B. Umsatz zwischen 60 % und 90 %).

Während des Betriebs der Katalysatoranordnung bildet sich ein Temperaturprofil aus. Bevorzugt ist dabei, dass das Temperaturprofil derart ist, dass bei der Gasphasenoxidation von aromatischen Kohlenwasserstoffen die Maximaltemperatur in der ersten Katalysatorlage um 10 bis 100 °C, mehr bevorzugt 20 bis 90 °C, am meis ten bevorzugt 30 bis 70 °C niedriger ist als in der zweiten Katalysatorlage.

Ist eine dritte Katalysatorlage vorhanden, so ist die intrinsische Aktivität der dritten Katalysatorlage vorzugsweise höher, als die der zweiten Katalysatorlage. Vorzugsweise entsteht während der Reaktion ein von der zweiten Katalysatorlage zur dritten Katalysatorlage abfallendes Temperaturprofil. Insbesondere ist das Temperaturprofil derart, dass bei der Gasphasenoxidation von Kohlenwasserstoffen die Maximaltemperatur in der dritten Katalysatorlage um 10 bis 100 °C, mehr bevorzugt 20 bis 90 °C, am m eisten bevorzugt 30 bis 70 °C niedriger ist als in der zweiten Katalysatorlage.

Ist eine vierte Katalysatorlage vorhanden, so ist die intrinsische Aktivität der vierten Katalysatorlage vorzugsweise höher, als die der dritten Katalysatorlage. Dabei ist vorzugsweise das Temperaturprofil von der zweiten zur vierten (und auch zur dritten) Katalysatorlage abfallend ($T_2 > T_3 > T_4$, jeweils bezogen auf die Maximaltemperatur in jeder Lage). Insbesondere ist das Temperaturprofil derart, dass bei der Gasphasenoxidation von Kohlenwasserstoffen die Maximaltemperatur in der vierten Katalysatorlage um 10 bis 100 °C, mehr bevorzugt 20 bis 90 °C, am meisten bevorzugt 30 bis 70 °C niedriger ist als in der zweiten und um 1 bis 50 °C, mehr bevorzugt um 5 bis 25°C, am meisten bev orzugt um 5 bis 10°C niedriger ist als in der dritten Katalysatorlage.

Bevorzugt ist insbesondere, dass die intrinsische Aktivität von der ersten zur zweiten Katalysatorlage abfällt und dann von der zweiten zur vierten Katalysatorlage ($A_1 > A_2 < A_3 < A_4$, mit A = intrinsische Aktivität der Aktivmasse in der jeweiligen Lage) ansteigt. Weiterhin bevorzugt ist, dass das Temperaturprofil von der ersten zur zweiten Katalysatorlage ansteigt, von der zweiten zur vierten Katalysatorlage abfällt ($T_1 < T_2 > T_3 > T_4$, jeweils bezogen auf die Maximaltemperatur in jeder Lage) und sich ein Temperaturmaximum in der zweiten Katalysatorlage ausbildet.

Die Katalysatorkörper bestehen aus einem inerten Trägerkörper und einer darauf aufgebrachten Aktivmasse. Die Katalysatorkörper der Katalysatorlagen werden in der üblichen Weise hergestellt, wobei eine dünne Schicht der Aktivmasse auf den inerten Trägerkörper aufgebracht wird. Dazu kann beispielsweise eine Suspension der Aktivmasse oder eine Lösung bzw. Suspension von Vorläuferverbindungen, die in die Komponenten der Aktivmasse überführt werden können, auf den inerten Träger aufgesprüht werden. Dies kann beispielsweise bei einer Temperatur von 80 bis 200 °C im Fli eßbett erfolgen. Die Aktivmasse kann aber beispielsweise auch in einer Art Dragiertrommel auf den inerten Träger aufgebracht werden.

Für den Beschichtungsvorgang wird die wässrige Lösung oder Suspension von Aktivkomponenten und eines organischen Binders, bevorzugterweise einem Co-Polymer aus Vinylacetat/Vinyllaurat, Vinylacetat/Ethylen oder Styrol/Acrylat, über eine oder mehrere Düsen auf den erwärmten, fluidisierten Träger aufgesprüht. Besonders günstig ist es, die Sprühflüssigkeit am Ort der höchsten Produktgeschwindigkeit aufzugeben, wodurch sich der Sprühstoff gleichmäßig im Bett verteilen kann. Der Sprühvorgang wird solange fortgeführt, bis entweder die Suspension verbraucht oder die erforderliche Menge an Aktivkomponenten auf dem Träger aufgebracht ist. Unter Aktivkomponenten werden Komponente der Aktivmasse verstanden, insbesondere in der Aktivmasse enthaltene Metallverbindungen. Die Aktivkomponenten können als Oxide oder in Form von Vorläuferverbindungen eingesetzt werden. Unter Vorläuferverbindungen werden Verbindungen verstanden, die sich beispielsweise durch Erhitzen an Luft in die Komponenten der Aktivmasse, also die Oxide, überführen lassen. Geeignete Vorläuferverbindungen sind beispielsweise Nitrate, Sulfate, Karbonate, Acetate oder Chloride der Metalle.

Nach einer Ausführungsform wird die Aktivmasse im Fließbett oder Wirbelbett unter Beihilfe geeigneter Bindemittel aufgebracht, so dass ein Schalenkatalysator erzeugt wird. Geeignete Bindemittel umfassen dem Fachmann geläufige organische Binder, bevorzugt Co-Polymere, vorteilhaft in Form einer wässrigen Suspensionen von Vinylacetat/Vinyllaurat, Vinylacetat/Acrylat, Styrol, Acrylat, Vinylacetat/Maleat sowie Vinylacetat/Ethylen. Besonders bevorzugt wird ein organischer polymerer oder co-polymerer Kleber, insbesondere ein Vinylacetat-Copolymer-Kleber als Bindemittel verwendet. Das verwendete Bindemittel wird in den üblichen Mengen der Aktivmasse zugegeben, beispielsweise mit etwas 10 bis 20 Gew.-% bezogen auf den Feststoffgehalt der Aktivmasse. Beispielhaft wird auf die EP 744214 verwiesen.

Die Bestimmung des Binderanteils wird durchgeführt, indem die beschichteten Katalysatorkörper bei 450 °C für 7 h kalziniert werden, wobei sich der organische Binder vollständig thermisch zersetzt. Der Binderanteil wird im Anschluss an die Kalzinierung nach Gl. 6 bestimmt:

Gl. 6:

$$A_B = \frac{M_E - M_A}{M_E} * 100\%$$

$A_B$ = Binderanteil
$M_E$ = Einwaage Katalysator vor Kalzinierung
$M_A$ = Auswaage Katalysator nach Kalzinierung

Die physikalisch-chemische Charakterisierung der Aktivmasse (BET, chemische Analytik) wird durchgeführt, indem nach der thermischen Zersetzung des Binders, die Aktivmasse mechanisch mittels eines Siebes von den Trägerringen abgetrennt wird. Der restliche, noch an den Trägerringen anhaftende Teil der Aktivmasse wurde durch Ultraschallbehandlung vollständig entfernt. Abschließend wurden die gewaschenen Trägerringe bei 120 °C in einem Trockenschrank getrocknet und gewogen. Der Anteil der Aktivmasse wird im Anschluss nach Gl. 7 bestimmt:

Gl. 7:

$$A_A = \frac{M_A - M_T}{M_A} * 100\%$$

$A_A$ = Anteil Aktivmasse
$M_A$ = Auswaage Katalysator nach Kalzinierung
$M_T$ = Auswaage Trägerringe

Die Bestimmung der spezifischen Oberfläche der Materialien erfolgt nach der BET-Methode gemäss DIN 66131; eine Veröffentlichung der BET-Methode findet sich auch in J. Am. Chem. Soc. 60, 309 (1938). Die zu bestimmende Probe wurde in einem Quarzrohr bei 350 °C unter Vakuum getrocknet (F = 50 ml(min) für 1,5 h). Der Reaktor wurde dann auf Raumtemperatur abgekühlt, evakuiert und in ein Dewar-Gefäß mit flüssigem Stickstoff getaucht. Die Stickstoff-Adsorption wurde bei 77 K mit einem RXM 100 Sorptionssystem (Advanced Scientific Design, Inc.) durchgeführt.

**Beispiele**

Es wurden katalytische Messungen an 4-Lagen-Katalysatoranordnungen aus Katalysatorkörpern durchgeführt. Zur Synthese der Katalysatorkörper wurden zwei verschiedene Typen Steatitringe als Formkörper mit der Bezeichnung Ring 8x6x5 und Ring 6x5x4 verwendet. Die Nomenklatur der geometrischen Abmessungen der Ringe entspricht Außendurchmesser (Da) [mm] x Höhe (H) [mm] x Innendurchmesser (Di) [mm]. Die geometrischen Abmessungen der unbeschichteten Formkörper ergeben sich aus Tabelle 2. Die unbeschichteten Formkörper wurden in eine Beschichtungsapparatur eingebracht und homogen mit der Aktivmasse beschichtet. Während des Beschichtungsvorgangs wird eine wässrige Suspension der Aktivkomponenten und eines organischen Binders über mehrere Düsen auf den erwärmten, fluidisierten Träger aufgesprüht, bis sich eine Aktivmasseschicht von ca. 50 - 150 μm ausgebildet hat. Tabelle 3 zeigt eine Übersicht über die verwendeten Katalysatorkörper und die jeweilige Zusammensetzung der Aktivmasse.

Zur Ausbildung der Katalysatorlagen wurden die jeweiligen Katalysatorkörper in ein salzbadgekühltes Rohr mit 25 mm innerem Durchmesser und 4 m Länge gefüllt. Im Rohr befand sich zentrisch angeordnet eine 3 mm Thermohülse mit eingebautem Zugelement zur Temperaturmessung.

Zur Durchführung der katalytischen Messung wurde durch das Rohr stündlich von oben nach unten etwa 3,7 bis 4,0 $Nm^3$ (Normkubikmeter) Luft mit einer Beladung von 30 bis 100 g ortho-Xylol/$Nm^3$ Luft (Reinheit ortho-Xylol > 98 %) bei einem Gesamtdruck von ca. 1500 mbar geleitet. Die Messungen wurden jeweils bei einer Beladung von etwa 40 bis 100 g ortho-Xylol/$Nm^3$ Luft und einer Salzbadtemperaturen zwischen 350 und 390 °C durchgeführt.

Die Phthalsäureanhydrid-Ausbeute wurde mit Gl. 8 berechnet:

$$\text{Gl. 8:} \qquad Y_{PA} = \left[ 139{,}52 - \left( 800 * \frac{A+B}{E} \right) + (100 - F) - (1{,}25 * H) - (1{,}1 * G) \right]$$

A = $CO_2$ im Produktstrom [Vol.-%]

B = CO im Produktstrom [Vol.-%]

H = Maleinsäureanhydrid im Produktstrom [Gew.-%]

E = ortho-Xylol-Beladung im Eduktstrom [g/$Nm^3$/h/Rohr]

F = ortho-Xylol-Reinheit des eingesetzten ortho-Xylols [Gew.-%]

G = ortho-Xylol-Schlupf in Bezug auf das gesamte eingesetzte ortho-Xylol [Gew.-%]

$Y_{PA}$ = Ausbeute Phthalsäureanhydrid (PSA) bezogen auf das Gesamtgewicht des eingesetzten ortho-Xylols [Gew.-%]

Wie aus Gl. 8 ersichtlich, ist die Phthalsäureanhydrid-Ausbeute unmittelbar abhängig von der Bildung der drei wichtigsten Nebenprodukte CO, $CO_2$ und Maleinsäureanhydrid.

**Tabelle 2: Geometrische Abmessungen und Eigenschaften der unbeschichteten Formkörper**

|  | Ring 8x6x5 | Ring 6x5x4 |
|---|---|---|
| Außendurchmesser x Höhe [$cm^2$] | 0,48 | 0,30 |
| Volumen [$cm^3$] | 0,184 | 0,079 |
| Oberfläche [$cm^2$] | 3,063 | 1,885 |
| Oberfläche/Volumen [$cm^{-1}$] | 16,7 | 24,0 |
| Volumen/Oberfläche [cm] | 0,060 | 0,042 |
| scheinbare Dichte [g/$cm^3$] | 2,61 | 2,61 |

**Tabelle 3: Verwendete Katalysatorkörper**

| Bezeichnung | Ringform | Binderanteil | Anteil Aktivmasse | BET | $TiO_2$ | $V_2O_5$ | Promotoren |
|---|---|---|---|---|---|---|---|
| | $(Da \times H \times Di)^1$ [mm] | [Gew.-%]$^3$ | [Gew.-%]$^2$ | [m$^2$/g] | [Gew.-%]$^3$ | [Gew.-%]$^3$ | [Gew.-%]$^4$ |
| Vergleichstest 1 | | | | | | | |
| A0 | 8x6x5 | 2,3 | 8,5 | 18 | 87,4 | 7,5 | 5,1 |
| A1 | 8x6x5 | 2,3 | 8,5 | 18 | 87,4 | 7,5 | 5,1 |
| A2 | 8x6x5 | 2,3 | 8,0 | 18 | 89,0 | 7,5 | 3,5 |
| A3 | 8x6x5 | 2,3 | 8,0 | 27 | 90,6 | 9,0 | 0,4 |
| Vergleichstest 2 | | | | | | | |
| B0 | 8x6x5 | 2,4 | 8,3 | 19 | 87,4 | 7,5 | 5,1 |
| B1 | 8x6x5 | 2,4 | 8,6 | 18 | 87,3 | 7,6 | 5,1 |
| B2.1 | 8x6x5 | 2,4 | 8,0 | 18 | 89,1 | 7,4 | 3,5 |
| B3.1 | 8x6x5 | 2,2 | 7,9 | 26 | 90,6 | 9,1 | 0,4 |
| Vergleichstest 3 | | | | | | | |
| B0 | 8x6x5 | 2,4 | 8,3 | 19 | 87,4 | 7,5 | 5,1 |
| C1 | 8x6x5 | 1,3 | 3,1 | 17 | 88,0 | 7,7 | 4,3 |
| B2.1 | 8x6x5 | 2,4 | 8,0 | 18 | 89,1 | 7,4 | 3,5 |
| B3.2 | 8x6x5 | 2,4 | 7,9 | 25 | 90,2 | 9,4 | 0,4 |
| Erfindungsgemäßer Test 1 | | | | | | | |
| B0 | 8x6x5 | 2,4 | 8,3 | 19 | 87,4 | 7,5 | 5,1 |
| D1 | 8x6x5 | 2,3 | 5,2 | 27 | 83,6 | 10,7 | 5,7 |
| B2.2 | 8x6x5 | 2,4 | 8,0 | 18 | 88,0 | 7,4 | 3,5 |
| B3.1 | 8x6x5 | 2,2 | 7,9 | 26 | 90,6 | 9,1 | 0,4 |
| Erfindungsgemäßer Test 2 | | | | | | | |
| B0 | 8x6x5 | 2,4 | 8,3 | 19 | 87,4 | 7,5 | 5,1 |
| E1 | 6x5x4 | 2,2 | 5,3 | 24 | 83,7 | 10,7 | 5,6 |
| B2.1 | 8x6x5 | 2,4 | 8,0 | 18 | 89,1 | 7,4 | 3,5 |

(fortgesetzt)

| Bezeichnung | Ringform | Binderanteil | Anteil Aktivmasse | BET | TiO$_2$ | V$_2$O$_5$ | Promotoren |
|---|---|---|---|---|---|---|---|
| | (Da x H x Di)[1] [mm] | [Gew.-%][3] | [Gew.-%][2] | [m$^2$/g] | [Gew.-%][3] | [Gew.-%][3] | [Gew.-%][4] |
| B3.2 | 8x6x5 | 2,4 | 7,9 | 25 | 90,2 | 9,4 | 0,4 |
| [1] Da = Außendurchmesser, H = Höhe, Di = Innendurchmesser<br>[2] Bezogen auf das Gesamtgewicht des Katalysatorkörpers<br>[3] Bezogen auf das Gesamtgewicht der Aktivmasse<br>[4] Vorwiegend Sb$_2$O$_3$ neben geringeren Anteilen, Nb$_2$O$_5$, P und Cs | | | | | | | |

**Tabelle 4: Füllparameter des Vergleichstests 1**

| Katalysatorlage x | Katalysator | Füllhöhen $L_x$ | Schüttdichte | scheinbare Dichte | Oberfläche / Volumen (Inertkörpers) | Ringform | $OF_A / V_x$ |
|---|---|---|---|---|---|---|---|
| | | [cm] | [g/cm$^3$] | [g/cm$^3$] | [cm$^{-1}$] | (Da x H x Di) [mm] | [cm$^{-1}$] |
| 1 | A0 | 40,9 | 0,92 | 2,623 | 16,67 | 8x6x5 | 14026 |
| 2 | A1 | 160,0 | 0,91 | 2,664 | 16,67 | 8x6x5 | 13947 |
| 3 | A2 | 60,9 | 0,91 | 2,622 | 16,67 | 8x6x5 | 13104 |
| 4 | A3 | 59,5 | 0,91 | 2,663 | 16,67 | 8x6x5 | 19667 |

**Tabelle 5: Füllparameter des Vergleichstests 2**

| Katalysatorlage x | Katalysator | Füllhöhen $L_x$ | Schüttdichte | scheinbare Dichte | Oberfläche / Volumen (Inertkörpers) | Ringform | $OF_A / V_x$ |
|---|---|---|---|---|---|---|---|
| | | [cm] | [g/cm$^3$] | [g/cm$^3$] | [cm$^{-1}$] | (Da x H x Di) [mm] | [cm$^{-1}$] |
| 1 | B0 | 40,0 | 0,89 | 2,624 | 16,67 | 8x6x5 | 14089 |
| 2 | B1 | 135,3 | 0,91 | 2,610 | 16,67 | 8x6x5 | 14065 |
| 3 | B2.1 | 60,5 | 0,90 | 2,619 | 16,67 | 8x6x5 | 12898 |
| 4 | B3.1 | 64,9 | 0,92 | 2,643 | 16,67 | 8x6x5 | 18884 |

**Tabelle 6: Füllparameter des Vergleichstests 3**

| Katalysatorlage x | Katalysator | Füllhöhen $L_x$ | Schüttdichte | scheinbare Dichte | Oberfläche / Volumen (Inertkörpers) | Ringform | $OF_A / V_x$ |
|---|---|---|---|---|---|---|---|
| | | [cm] | [g/cm$^3$] | [g/cm$^3$] | [cm$^{-1}$] | (Da x H x Di) [mm] | [cm$^{-1}$] |
| 1 | B0 | 40,3 | 0,89 | 2,624 | 16,67 | 8x6x5 | 14089 |
| 2 | C1 | 155,0 | 0,90 | 2,610 | 16,67 | 8x6x5 | 4844 |
| 3 | B2.1 | 60,5 | 0,90 | 2,619 | 16,67 | 8x6x5 | 12898 |
| 4 | B3.2 | 65,0 | 0,92 | 2,620 | 16,67 | 8x6x5 | 18415 |

**Tabelle 7: Füllparameter des erfindungsgemäßen Tests 1**

| Katalysatorlage x | Katalysator | Füllhöhen $L_x$ | Schüttdichte | scheinbare Dichte | Oberfläche / Volumen (Inertkörpers) | Ringform | $OF_A / V_x$ |
|---|---|---|---|---|---|---|---|
| | | [cm] | [g/cm³] | [g/cm³] | [cm⁻¹] | (Da x H x Di [mm] | [cm⁻¹] |
| 1 | B0 | 41,2 | 0,89 | 2,624 | 16,67 | 8x6x5 | 14089 |
| 2 | D1 | 154,5 | 0,90 | 2,600 | 16,67 | 8x6x5 | 12492 |
| 3 | B2.2 | 60,7 | 0,88 | 2,573 | 16,67 | 8x6x5 | 12692 |
| 4 | B3.1 | 65,4 | 0,92 | 2,643 | 16,67 | 8x6x5 | 18884 |

**Tabelle 8: Füllparameter des erfindungsgemäßen Tests 2**

| Katalysatorlage x | Katalysator | Füllhöhen $L_x$ [cm] | Schüttdichte [g/cm³] | scheinbare Dichte [g/cm³] | Oberfläche / Volumen (Inertkörpers) [cm⁻¹] | Ringform (Da x H x Di) [mm] | $OF_A / V_x$ [cm⁻¹] |
|---|---|---|---|---|---|---|---|
| 1 | B0 | 39,7 | 0,89 | 2,624 | 16,7 | 8x6x5 | 14089 |
| 2 | E1 | 155,3 | 0,96 | 2,693 | 24,0 | 6x5x4 | 12350 |
| 3 | B2.1 | 60,9 | 0,90 | 2,619 | 16,7 | 8x6x5 | 12898 |
| 4 | B3.2 | 64,3 | 0,92 | 2,620 | 16,7 | 8x6x5 | 18415 |

**Tabelle 9: Katalysatorperformance des Vergleichstests 1**

| TOS | Volumenstrom Luft | Beladung ortho-Xylol | SBT | CO | $CO_2$ | MSA | ortho-Xylol | Ausbeute PSA | $(CO+CO_2)$/ Beladung |
|---|---|---|---|---|---|---|---|---|---|
| [h] | [Nm³] | [g/Nm³/h/Rohr] | [°C] | [Vol.-%][1] | [Vol.-%][1] | [Gew.-%][2] | [Gew.-%][2] | [Gew.-%][3] | [Vol.-%/g/Nm³/h/Rohr] |
| 178,6 | 4,0 | 33,7 | 379 | 0,337 | 0,781 | 5,0 | 0,01 | 108,7 | 0,0332 |
| 203,4 | 4,0 | 35,7 | 378 | 0,357 | 0,827 | 5,1 | 0,01 | 108,7 | 0,0332 |
| 705,1 | 4,0 | 46,3 | 371 | 0,465 | 1,115 | 4,9 | 0,02 | 108,0 | 0,0341 |
| 728,7 | 4,0 | 48,3 | 370 | 0,487 | 1,193 | 5,0 | 0,02 | 107,5 | 0,0348 |
| 752,2 | 4,0 | 48,3 | 370 | 0,490 | 1,137 | 5,0 | 0,02 | 108,3 | 0,0337 |
| 2821,4 | 4,0 | 79,9 | 359 | 0,749 | 1,785 | 4,5 | 0,03 | 110,5 | 0,0317 |
| 2847,0 | 4,0 | 79,9 | 360 | 0,752 | 1,798 | 4,5 | 0,03 | 110,3 | 0,0319 |
| 2867,2 | 4,0 | 81,4 | 360 | 0,757 | 1,810 | 4,5 | 0,03 | 110,7 | 0,0315 |

**Tabelle 10: Katalysatorperformance des Vergleichstests 2**

| TOS | Volumenstrom Luft | Beladung ortho-Xylol | SBT | CO | $CO_2$ | MSA | ortho-Xylol | Ausbeute PSA | $(CO+CO_2)/$ Beladung |
|---|---|---|---|---|---|---|---|---|---|
| [h] | [$Nm^3$] | [g/$Nm^3$/h/ Rohr] | [°C] | [Vol.-%][1] | [Vol.-%][1] | [Gew.-%][2] | [Gew.-%][2] | [Gew.-%][3] | [Vol.-%/g/$Nm^3$/h/ Rohr] |
| 254,3 | 3,7 | 73,1 | 368 | 0,758 | 1,712 | 4,9 | 0,02 | 108,4 | 0,0338 |
| 278,4 | 3,7 | 73,1 | 368 | 0,732 | 1,671 | 4,7 | 0,02 | 109,3 | 0,0328 |
| 302,5 | 3,7 | 73,1 | 368 | 0,726 | 1,670 | 4,7 | 0,02 | 109,5 | 0,0328 |
| 538,1 | 3,7 | 82,0 | 364 | 0,806 | 1,866 | 4,7 | 0,02 | 109,6 | 0,0326 |
| 562,1 | 3,7 | 83,9 | 363 | 0,820 | 1,958 | 4,7 | 0,03 | 109,2 | 0,0331 |
| 761,3 | 3,7 | 90,9 | 356 | 0,848 | 2,048 | 4,3 | 0,02 | 110,6 | 0,0319 |
| 785,3 | 3,7 | 90,9 | 356 | 0,848 | 2,056 | 4,3 | 0,03 | 110,6 | 0,0320 |
| 809,3 | 3,7 | 90,9 | 356 | 0,838 | 2,038 | 4,2 | 0,03 | 110,9 | 0,0316 |

**Tabelle 11: Katalysatorperformance des Vergleichstests 3**

| TOS | Volumenstrom Luft | Beladung ortho-Xylol | SBT | CO | $CO_2$ | MSA | ortho-Xylol | Ausbeute PSA | (CO+$CO_2$)/ Beladung |
|---|---|---|---|---|---|---|---|---|---|
| [h] | [$Nm^3$] | [g/$Nm^3$/h/Rohr] | [°C] | [Vol.-%][1] | [Vol.-%][1] | [Gew.-%][2] | [Gew.-%][2] | [Gew.-%][3] | [Vol.-%/g/$Nm^3$/h/Rohr] |
| | | | | | | | | | |
| 487,3 | 4,0 | 41,6 | 380 | 0,394 | 0,908 | 3,8 | 0,01 | 111,8 | 0,0313 |
| 580,4 | 4,0 | 52,1 | 372 | 0,464 | 1,147 | 3,6 | 0,02 | 112,3 | 0,0309 |
| 620,0 | 4,0 | 54,1 | 371 | 0,490 | 1,160 | 4,2 | 0,02 | 111,9 | 0,0305 |
| 938,0 | 4,0 | 54,1 | 364 | 0,509 | 1,199 | 4,0 | 0,02 | 111,3 | 0,0315 |
| 962,0 | 4,0 | 54,1 | 364 | 0,504 | 1,180 | 4,0 | 0,02 | 111,7 | 0,0311 |
| 1051,9 | 4,0 | 50,6 | 361 | 0,467 | 1,092 | 3,9 | 0,03 | 112,0 | 0,0308 |
| 1062,0 | 4,0 | 50,6 | 360 | 0,469 | 1,097 | 4,0 | 0,03 | 111,7 | 0,0309 |

**Tabelle 12: Katalysatorperformance des erfindungsgemäßen Tests 1**

| TOS | Volumenstrom Luft | Beladung ortho-Xylol | SBT | CO | $CO_2$ | MSA | ortho-Xylol | Ausbeute PSA | $(CO+CO_2)/$ Beladung |
|---|---|---|---|---|---|---|---|---|---|
| [h] | $[Nm^3]$ | $[g/Nm^3/h/Rohr]$ | [°C] | [Vol.-%][1] | [Vol.-%][1] | [Gew.-%][2] | [Gew.-%][2] | [Gew.-%][3] | $[Vol.-\%/g/Nm^3/h/Rohr]$ |
| 647,4 | 4,0 | 68,0 | 378 | 0,604 | 1,390 | 4,1 | 0,02 | 112,9 | 0,0293 |
| 703,2 | 4,0 | 69,9 | 375 | 0,613 | 1,420 | 4,2 | 0,03 | 113,0 | 0,0291 |
| 742,4 | 4,0 | 71,0 | 373 | 0,599 | 1,393 | 4,1 | 0,02 | 113,9 | 0,0280 |
| 766,4 | 4,0 | 71,0 | 373 | 0,610 | 1,422 | 4,1 | 0,02 | 113,5 | 0,0286 |
| 799,1 | 4,0 | 72,1 | 372 | 0,573 | 1,342 | 4,2 | 0,03 | 115,0 | 0,0265 |
| 823,0 | 4,0 | 74,0 | 371 | 0,602 | 1,444 | 4,2 | 0,03 | 114,2 | 0,0276 |
| 847,6 | 4,0 | 76,0 | 370 | 0,634 | 1,517 | 4,2 | 0,03 | 113,6 | 0,0283 |
| 878,0 | 4,0 | 77,9 | 369 | 0,640 | 1,525 | 4,2 | 0,03 | 114,0 | 0,0278 |
| 903,0 | 4,0 | 80,1 | 368 | 0,649 | 1,483 | 4,2 | 0,02 | 115,0 | 0,0266 |
| 991,1 | 4,0 | 80,9 | 365 | 0,645 | 1,537 | 3,9 | 0,03 | 115,0 | 0,0270 |
| 1037,5 | 4,0 | 80,9 | 362 | 0,625 | 1,493 | 3,9 | 0,04 | 115,6 | 0,0262 |
| 1057,3 | 4,0 | 80,9 | 361 | 0,635 | 1,580 | 3,8 | 0,05 | 114,8 | 0,0274 |
| 1080,1 | 4,0 | 81,5 | 360 | 0,636 | 1,527 | 3,7 | 0,02 | 115,6 | 0,0266 |
| 1102,8 | 4,0 | 81,5 | 360 | 0,628 | 1,514 | 3,7 | 0,02 | 115,8 | 0,0263 |
| 1186,0 | 4,0 | 76,2 | 360 | 0,000 | 0,000 | 3,7 | 0,02 | 136,9 | 0,0000 |
| 1212,0 | 4,0 | 81,2 | 360 | 0,598 | 1,433 | 3,8 | 0,02 | 116,7 | 0,0250 |
| 1224,7 | 4,0 | 81,2 | 360 | 0,601 | 1,458 | 3,8 | 0,04 | 116,4 | 0,0254 |
| 1247,5 | 4,0 | 81,2 | 360 | 0,602 | 1,447 | 3,8 | 0,04 | 116,6 | 0,0252 |
| 1270,3 | 4,0 | 81,2 | 360 | 0,602 | 1,447 | 3,8 | 0,04 | 116,6 | 0,0252 |
| 1354,1 | 4,0 | 81,2 | 354 | 0,585 | 1,428 | 3,5 | 0,12 | 117,2 | 0,0248 |

**Tabelle 13: Katalysatorperformance des erfindungsgemäßen Tests 2**

| TOS | Volumenstrom Luft | Beladung ortho-Xylol | SBT | CO | $CO_2$ | MSA | ortho-Xylol | Ausbeute PSA | (CO+$CO_2$)/ Beladung |
|---|---|---|---|---|---|---|---|---|---|
| [h] | [$Nm^3$] | [g/$Nm^3$/h/Rohr] | [°C] | [Vol.-%][1] | [Vol.-%][1] | [Gew.-%][2] | [Gew.-%][2] | [Gew.-%][3] | [Vol.-%/g/$Nm^3$/h/Rohr] |
| 156,2 | 4,0 | 43,0 | 387 | 0,463 | 1,019 | 4,6 | 0,01 | 108,2 | 0,0345 |
| 180,4 | 4,0 | 47,0 | 384 | 0,508 | 1,115 | 4,7 | 0,01 | 108,0 | 0,0345 |
| 204,3 | 4,0 | 51,0 | 381 | 0,560 | 1,230 | 4,8 | 0,01 | 107,5 | 0,0351 |
| 229,5 | 4,0 | 55,9 | 377 | 0,595 | 1,317 | 4,7 | 0,01 | 108,3 | 0,0342 |
| 250,5 | 4,0 | 59,9 | 373 | 0,581 | 1,318 | 4,3 | 0,02 | 110,7 | 0,0317 |
| 274,5 | 4,0 | 59,9 | 373 | 0,587 | 1,332 | 4,3 | 0,02 | 110,5 | 0,0320 |
| 298,5 | 4,0 | 59,9 | 373 | 0,585 | 1,335 | 4,3 | 0,02 | 110,5 | 0,0321 |
| 348,5 | 4,0 | 66,1 | 367 | 0,578 | 1,408 | 3,9 | 0,03 | 112,6 | 0,0300 |
| 1260,8 | 4,0 | 78,0 | 357 | 0,636 | 1,585 | 3,3 | 0,08 | 114,5 | 0,0285 |
| 1284,9 | 4,0 | 78,0 | 357 | 0,633 | 1,539 | 3,2 | 0,07 | 115,2 | 0,0279 |
| 1309,0 | 4,0 | 78,0 | 357 | 0,623 | 1,511 | 3,2 | 0,07 | 115,6 | 0,0274 |
| 1335,1 | 4,0 | 80,9 | 357 | 0,636 | 1,587 | 3,2 | 0,09 | 115,5 | 0,0275 |
| 1383,6 | 4,0 | 84,9 | 356 | 0,676 | 1,650 | 3,5 | 0,10 | 115,2 | 0,0274 |
| 1406,4 | 4,0 | 84,9 | 355 | 0,681 | 1,663 | 3,5 | 0,09 | 115,0 | 0,0276 |
| 1551,6 | 4,0 | 84,9 | 355 | 0,677 | 1,731 | 3,5 | 0,13 | 114,4 | 0,0284 |
| 1578,4 | 4,0 | 84,9 | 355 | 0,648 | 1,747 | 3,5 | 0,12 | 114,5 | 0,0282 |
| 1621,3 | 4,0 | 84,9 | 355 | 0,674 | 1,717 | 3,5 | 0,13 | 114,5 | 0,0282 |

[1] Bezogen auf das Gesamtvolumen des Produktstroms
[2] Bezogen auf das Gesamtgewicht des Produktstroms
[3] Bezogen auf das Gesamtgewicht des eingesetzten ortho-Xylols

**Patentansprüche**

1. Katalysatoranordnung zur Herstellung von Phthalsäureanhydrid durch Gasphasenoxidation aromatischer Kohlenwasserstoffe, umfassend einen Reaktor mit einer Gaseintrittsseite für ein Eduktgas und einer Gasaustrittsseite für ein Produktgas, sowie einer ersten Katalysatorlage aus Katalysatorkörpern, und mindestens einer zweiten Katalysatorlage aus Katalysatorkörpern, wobei die erste Katalysatorlage an der Gaseintrittsseite und die zweite Katalysatorlage nachfolgend zur ersten Katalysatorlage in Gasdurchflussrichtung angeordnet ist und die Katalysatorkörper eine äußere Schicht Aktivmasse aufweisen, **dadurch gekennzeichnet, dass** in der ersten Katalysatorlage und/oder in der zweiten Katalysatorlage der Aktivmassegehalt, bezogen auf das Gesamtgewicht der Katalysatorkörper, unter 7 Gew.-% beträgt und in der jeweiligen Katalysatorlage das Verhältnis der Gesamtoberfläche der Aktivmasse zum Volumen der Katalysatorlage 10000 cm$^{-1}$ bis 20000 cm$^{-1}$, bevorzugt 12000 cm$^{-1}$ bis 16000 cm$^{-1}$ beträgt.

2. Katalysatoranordnung nach Anspruch 1, **dadurch gekennzeichnet, dass** der Aktivmassegehalt, bezogen auf das Gesamtgewicht der Katalysatorkörper, der ersten und/oder der zweiten Katalysatorlage unter 6 Gew.-%, besonders bevorzugt unter 5 Gew.-% oder noch stärker bevorzugt unter 4 Gew.-% beträgt.

3. Katalysatoranordnung nach einem der vorhergehenden Ansprüche, **dadurch gekennzeichnet, dass** in der ersten Katalysatorlage das Verhältnis der Gesamtoberfläche der Aktivmasse zum Volumen der Katalysatorlage von dem der zweiten Katalysatorlage um weniger als 15 % abweicht, bevorzugterweise um weniger als 10 % abweicht.

4. Katalysatoranordnung nach einem der vorhergehenden Ansprüche, **dadurch gekennzeichnet, dass** die Katalysatorkörper der ersten Katalysatorlage eine höhere Aktivmassebeladung aufweisen als die Katalysatorkörper der zweiten Katalysatorlage, jeweils bezogen auf die Masse der Katalysatorkörper.

5. Katalysatoranordnung nach einem der vorhergehenden Ansprüche, **dadurch gekennzeichnet, dass** die Aktivmasse der Katalysatorkörper der ersten Katalysatorlage eine niedrigere BET-Oberfläche aufweist als die Aktivmasse der Katalysatorkörper der zweiten Katalysatorlage.

6. Katalysatoranordnung nach einem der vorhergehenden Ansprüche, **dadurch gekennzeichnet, dass** die Katalysatorkörper der ersten Katalysatorlage eine Aktivmasse mit einem auf die Masse der Aktivmasse bezogenen prozentual niedrigeren $V_2O_5$-Gehalt aufweisen als die Katalysatorkörper der zweiten Katalysatorlage.

7. Katalysatoranordnung nach einem der vorhergehenden Ansprüche, **dadurch gekennzeichnet, dass** die Katalysatorkörper der ersten Katalysatorlage eine Aktivmasse mit einem auf die Masse der Aktivmasse bezogenen prozentual niedrigeren Promotor-Gehalt aufweisen als die Katalysatorkörper der zweiten Katalysatorlage.

8. Katalysatoranordnung nach einem der vorstehenden Ansprüche, **dadurch gekennzeichnet, dass** mindestens eine dritte Katalysatorlage nachfolgend zur zweiten Katalysatorlage in Gasdurchflussrichtung angeordnet ist und in der dritten Katalysatorlage das Verhältnis der Gesamtoberfläche der Aktivmasse zum Volumen der Katalysatorlage von dem der ersten Katalysatorlage um weniger als 15 % abweicht, bevorzugterweise um weniger als 10 % abweicht.

9. Katalysatoranordnung nach Anspruch 8, **dadurch gekennzeichnet, dass** die Katalysatorkörper der zweiten Katalysatorlage eine niedrigere Aktivmassebeladung aufweisen als die Katalysatorkörper der dritten Katalysatorlage, jeweils bezogen auf die Masse der Katalysatorkörper.

10. Katalysatoranordnung nach einem der Ansprüche 8 oder 9, **dadurch gekennzeichnet, dass** die Katalysatorkörper der zweiten Katalysatorlage eine Aktivmasse mit einer höheren BET-Oberfläche aufweisen als die der dritten Katalysatorlage.

11. Katalysatoranordnung nach einem der Ansprüche 8 bis 10, **dadurch gekennzeichnet, dass** die Katalysatorkörper der zweiten Katalysatorlage eine Aktivmasse mit einem auf die Masse der Aktivmasse bezogenen prozentual höheren $V_2O_5$-Gehalt aufweisen als die der dritten Katalysatorlage.

12. Katalysatoranordnung nach einem der Ansprüche 8 bis 11, **dadurch gekennzeichnet, dass** die Katalysatorkörper der zweiten Katalysatorlage eine Aktivmasse mit einem auf die Masse der Aktivmasse bezogenen höheren Promotor-Gehalt aufweisen als die der dritten Katalysatorlage.

13. Katalysatoranordnung nach einem der vorhergehenden Ansprüche, **dadurch gekennzeichnet, dass** die Länge der ersten Katalysatorlage in Gasdurchflussrichtung 5 bis 25 % der Länge des Reaktors in Gasdurchflussrichtung beträgt.

14. Katalysatoranordnung nach einem der Ansprüche 8 bis 12, **dadurch gekennzeichnet, dass** die Länge der zweiten Katalysatorlage in Gasdurchflussrichtung 30 bis 60 % der Länge des Reaktors in Gasdurchflussrichtung beträgt.

15. Verfahren zur Herstellung von Phthalsäureanhydrid durch Gasphasenoxidation aromatischer Kohlenwasserstoffe, **dadurch gekennzeichnet, dass** ein Eduktgas, das einen aromatischen Kohlenwasserstoff enthält, durch eine Katalysatoranordnung nach einem der vorhergehenden Ansprüche geleitet wird.

16. Verwendung einer Katalysatoranordnung nach einem der Ansprüche 1 bis 14 zur Herstellung von Phthalsäureanhydrid.

**Claims**

1. Catalyst arrangement for preparing phthalic anhydride by gas-phase oxidation of aromatic hydrocarbons, which comprises a reactor having a gas inlet end for a feed gas and a gas outlet end for a product gas and also a first catalyst zone made up of catalyst bodies, and at least one second catalyst zone made up of catalyst bodies, where the first catalyst zone is arranged at the gas inlet end and the second catalyst zone is arranged downstream of the first catalyst zone in the gas flow direction and the catalyst bodies have an outer layer of active composition, **characterized in that** the active composition content in the first catalyst zone and/or in the second catalyst zone is less than 7% by weight, based on the total weight of the catalyst bodies, and the ratio of the total surface area of the active composition to the volume of the catalyst zone in the respective catalyst zone is from 10 000 $cm^{-1}$ to 20 000 $cm^{-1}$, preferably from 12 000 $cm^{-1}$ to 16 000 $cm^{-1}$.

2. Catalyst arrangement according to Claim 1, **characterized in that** the active composition content, based on the total weight of the catalyst bodies, of the first and/or second catalyst zone is less than 6% by weight, particularly preferably less than 5% by weight and even more preferably less than 4% by weight.

3. Catalyst arrangement according to either of the preceding claims, **characterized in that** the ratio of the total surface area of the active composition to the volume of the catalyst zone in the first catalyst zone differs from that in the second catalyst zone by less than 15%, preferably by less than 10%.

4. Catalyst arrangement according to any of the preceding claims, **characterized in that** the catalyst bodies of the first catalyst zone have a higher active composition loading than the catalyst bodies of the second catalyst zone, in each case based on the mass of the catalyst bodies.

5. Catalyst arrangement according to any of the preceding claims, **characterized in that** the active composition of the catalyst bodies of the first catalyst zone has a lower BET surface area than the active composition of the catalyst bodies of the second catalyst zone.

6. Catalyst arrangement according to any of the preceding claims, **characterized in that** the catalyst bodies of the first catalyst zone have an active composition having a lower percentage $V_2O_5$ content, based on the mass of the active composition, than the catalyst bodies of the second catalyst zone.

7. Catalyst arrangement according to any of the preceding claims, **characterized in that** the catalyst bodies of the first catalyst zone have an active composition having a lower percentage promoter content, based on the mass on the active composition, than the catalyst bodies of the second catalyst zone.

8. Catalyst arrangement according to any of the preceding claims, **characterized in that** at least one third catalyst zone is arranged downstream of the second catalyst zone in the gas flow direction and in the third catalyst zone the ratio of the total surface area of the active composition to the volume of the catalyst zone differs from that of the first catalyst zone by less than 15%, preferably by less than 10%.

9. Catalyst arrangement according to Claim 8, **characterized in that** the catalyst bodies of the second catalyst zone have a lower active composition loading than the catalyst bodies of the third catalyst zone, in each case based on the mass of the catalyst bodies.

**10.** Catalyst arrangement according to either Claim 8 or 9, **characterized in that** the catalyst bodies of the second catalyst zone have an active composition having a higher BET surface area than those of the third catalyst zone.

**11.** Catalyst arrangement according to any of Claims 8 to 10, **characterized in that** the catalyst bodies of the second catalyst zone have an active composition having a higher percentage $V_2O_5$ content, based on the mass of the active composition, than those of the third catalyst zone.

**12.** Catalyst arrangement according to any of Claims 8 to 11, **characterized in that** the catalyst bodies of the second catalyst zone have an active composition having a higher promoter content, based on the mass of the active composition, than those of the third catalyst zone.

**13.** Catalyst arrangement according to any of the preceding claims, **characterized in that** the length of the first catalyst zone in the gas flow direction is from 5 to 25% of the length of the reactor in the gas flow direction.

**14.** Catalyst arrangement according to any of Claims 8 to 12, **characterized in that** the length of the second catalyst zone in the gas flow direction is from 30 to 60% of the length of the reactor in the gas flow direction.

**15.** Process for preparing phthalic anhydride by gas-phase oxidation of aromatic hydrocarbons, **characterized in that** a feed gas containing an aromatic hydrocarbon is passed through a catalyst arrangement according to any of the preceding claims.

**16.** Use of a catalyst arrangement according to any of Claims 1 to 14 for preparing phthalic anhydride.


**Revendications**

**1.** Agencement catalytique pour la préparation d'anhydride d'acide phtalique par oxydation en phase gazeuse d'hydrocarbures aromatiques, comprenant un réacteur présentant un côté entrée de gaz, pour un gaz de départ, et côté sortie de gaz, pour un gaz produit, ainsi qu'une première couche catalytique constituée par des corps catalytiques et au moins une deuxième couche catalytique constituée par des corps catalytiques, la première couche catalytique étant disposée sur le côté entrée de gaz et la deuxième couche catalytique étant disposée en aval de la première couche catalytique dans le sens d'écoulement du gaz et les corps catalytiques présentant une couche externe de masse active, **caractérisé en ce que** la teneur en masse active dans la première couche catalytique et/ou dans la deuxième couche catalytique, par rapport au poids total des corps catalytiques, est inférieure à 7% en poids et, dans chaque couche catalytique, le rapport de la surface totale de la masse active au volume de la couche catalytique est de 10.000 cm$^{-1}$ à 20.000 cm$^{-1}$, de préférence de 12.000 cm$^{-1}$ à 16.000 cm$^{-1}$.

**2.** Agencement catalytique selon la revendication 1, **caractérisé en ce que** la teneur en masse active, par rapport au poids total des corps catalytiques, de la première et/ou de la deuxième couche catalytique est inférieure à 6% en poids, de manière particulièrement préférée inférieure à 5% en poids ou, de manière encore plus préférée, inférieure à 4% en poids.

**3.** Agencement catalytique selon l'une quelconque des revendications précédentes, **caractérisé en ce que**, dans la première couche catalytique, le rapport de la surface totale de la masse active au volume de la couche catalytique s'écarte de moins de 15%, de préférence de moins de 10%, de celui de la deuxième couche catalytique.

**4.** Agencement catalytique selon l'une quelconque des revendications précédentes, **caractérisé en ce que** les corps catalytiques de la première couche catalytique présentent une charge en masse active supérieure à celle des corps catalytiques de la deuxième couche catalytique, à chaque fois par rapport à la masse des corps catalytiques.

**5.** Agencement catalytique selon l'une quelconque des revendications précédentes, **caractérisé en ce que** la masse active des corps catalytiques de la première couche catalytique présentent une surface BET inférieure à celle de la masse active des corps catalytiques de la deuxième couche catalytique.

**6.** Agencement catalytique selon l'une quelconque des revendications précédentes, **caractérisé en ce que** les corps catalytiques de la première couche catalytique présentent une masse active présentant une teneur en $V_2O_5$, en % par rapport à la masse de la masse active, inférieure à celle des corps catalytiques de la deuxième couche catalytique.

7. Agencement catalytique selon l'une quelconque des revendications précédentes, **caractérisé en ce que** les corps catalytiques de la première couche catalytique présentent une masse active présentant une teneur en promoteur, en % par rapport à la masse de la masse active, inférieure à celle des corps catalytiques de la deuxième couche catalytique.

8. Agencement catalytique selon l'une quelconque des revendications précédentes, **caractérisé en ce qu'**au moins une troisième couche catalytique est disposée en aval de la deuxième couche catalytique dans le sens d'écoulement du gaz et, dans la troisième couche catalytique, le rapport de la surface totale de la masse active au volume de la couche catalytique s'écarte de moins de 15%, de préférence de moins de 10%, de celui de la première couche catalytique.

9. Agencement catalytique selon la revendication 8, **caractérisé en ce que** les corps catalytiques de la deuxième couche catalytique présentent une charge en masse active inférieure à celle des corps catalytiques de la troisième couche catalytique, à chaque fois par rapport à la masse des corps catalytiques.

10. Agencement catalytique selon l'une quelconque des revendications 8 ou 9, **caractérisé en ce que** les corps catalytiques de la deuxième couche catalytique présentent une masse active présentant une surface BET supérieure à celle de la troisième couche catalytique.

11. Agencement catalytique selon l'une quelconque des revendications 8 à 10, **caractérisé en ce que** les corps catalytiques de la deuxième couche catalytique présentent une masse active présentant une teneur en $V_2O_5$, en % par rapport à la masse de la masse active, supérieure à celle des corps catalytiques de la troisième couche catalytique.

12. Agencement catalytique selon l'une quelconque des revendications 8 à 11, **caractérisé en ce que** les corps catalytiques de la deuxième couche catalytique présentent une masse active présentant une teneur en promoteur, en % par rapport à la masse de la masse active, supérieure à celle des corps catalytiques de la troisième couche catalytique.

13. Agencement catalytique selon l'une quelconque des revendications précédentes, **caractérisé en ce que** la longueur de la première couche catalytique, dans le sens d'écoulement du gaz, représente 5 à 25% de la longueur du réacteur dans le sens d'écoulement du gaz.

14. Agencement catalytique selon l'une quelconque des revendications 8 à 12, **caractérisé en ce que** la longueur de la deuxième couche catalytique, dans le sens d'écoulement du gaz, représente 30 à 60% de la longueur du réacteur dans le sens d'écoulement du gaz.

15. Procédé pour la préparation d'anhydride de l'acide phtalique par oxydation en phase gazeuse d'hydrocarbures aromatiques, **caractérisé en ce qu'**un gaz de départ, qui contient un hydrocarbure aromatique, est guidé à travers un agencement catalytique selon l'une quelconque des revendications précédentes.

16. Utilisation d'un agencement catalytique selon l'une quelconque des revendications 1 à 14 pour la préparation d'anhydride de l'acide phtalique.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9961434 A1
- WO 9961433 A1
- WO 2004103944 A1
- WO 2007134849 A1
- WO 2011032658 A1
- WO 2006092304 A1
- WO 2008077791 A1
- EP 0985648 A1
- EP 744214 A

**In der Beschreibung aufgeführte Nicht-Patentliteratur**

- *J. Am. Chem. Soc.,* 1938, vol. 60, 309